(19) Europäisches Patentamt
European Patent Office
Office européen des brevets

(11) Veröffentlichungsnummer: **0 509 301 A1**

(12) **EUROPÄISCHE PATENTANMELDUNG**

(21) Anmeldenummer: **92105402.9**

(22) Anmeldetag: **28.03.92**

(51) Int. Cl.[5]: **C07D 209/08**, C07D 209/86, C07D 209/94, C07D 317/34, C07D 317/44, C07D 317/46, C07C 211/46, C07C 211/52, //C07D263/38,C07D263/52

(30) Priorität: **19.04.91 DE 4112841**

(43) Veröffentlichungstag der Anmeldung:
**21.10.92 Patentblatt 92/43**

(84) Benannte Vertragsstaaten:
**BE CH DE FR GB IT LI NL**

(71) Anmelder: **BASF Aktiengesellschaft**
**Carl-Bosch-Strasse 38**
**W-6700 Ludwigshafen(DE)**

(72) Erfinder: **Diehl, Klaus, Dr.**
**Luitpoldstrasse 24**
**W-6733 Hassloch(DE)**
Erfinder: **Fischer, Martin, Dr.**
**Elbinger Weg 1**
**W-6700 Ludwigshafen(DE)**
Erfinder: **Dimmler, Manfred, Dr.**
**Nordring 53**
**W-6701 Dannstadt-Schauernheim(DE)**

(54) **Verfahren zur Herstellung von Indoleninen.**

(57) Verfahren zur Herstellung von Indoleninen der allgemeinen Formel I

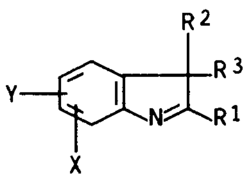

(I),

in der

$R^1$, $R^2$, $R^3$ unabhängig voneinander Wasserstoff, $C_1$- bis $C_{20}$-Alkyl, $C_2$- bis $C_{20}$-Alkenyl, $C_3$- bis $C_8$-Cycloalkyl, Aryl, $C_7$- bis $C_{20}$-Alkylphenyl, $C_7$- bis $C_{20}$-Phenalkyl oder $R^1$ und $R^2$ oder $R^1$ und $R^3$ gemeinsam eine gegebenenfalls durch $C_1$- bis $C_{12}$-Alkyl substituierte $C_3$- bis $C_8$-Alkylenkette,

X, Y unabhängig voneinander Wasserstoff, $C_1$- bis $C_8$-Alkyl, $C_1$- bis $C_8$-Alkoxy, $C_2$- bis $C_8$-Alkoxyalkyl, $C_2$- bis $C_8$-Alkoxycarbonyl, $C_1$- bis $C_4$-Alkylsulfonyl, Halogen, Nitro oder Cyano

bedeuten, durch Umsetzung von 4-Methylen-1,3-dioxolan-2-onen der allgemeinen Formel II

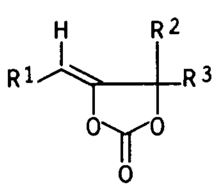

(II),

in der die Reste $R^1$, $R^2$ und $R^3$ die obengenannten Bedeutungen haben, mit Anilinen der allgemeinen Formel III

EP 0 509 301 A1

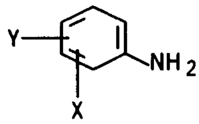

(III),

in der die Reste X und Y die obengenannten Bedeutungen haben, in Gegenwart von Lewissäuren und gegebenenfalls von Halogeniden der 1. oder 2. Hauptgruppe des Periodensystems der Elemente bei Temperaturen von 100 bis 350 °C und Drücken von 1 bis 50 bar, indem man die Umsetzung in polaren Lösungsmitteln durchführt.

Die vorliegende Erfindung betrifft ein verbessertes Verfahren zur Herstellung von Indoleninen aus 4-Methylen-1,3-dioxolan-2-onen und Anilinen bei erhöhten Temperaturen in Gegenwart eines Gemisches aus einer Lewissäure und einem Halogenid der 1. oder 2. Hauptgruppe des PSE, indem die Umsetzung in polaren Lösungsmitteln durchgeführt wird.

Aus der EP-A-8097 ist bekannt, daß man Indolenine in einer zweistufigen Heterogenreaktion aus 4-Methylen-1,3-dioxolan-2-onen über die Zwischenstufe 4-Methylen-1,3-oxazolidin-2-on erhält. Die Synthese der Indolenine kann zweistufig oder als Eintopfsynthese, ohne Isolierung der Oxazolidinone durchgeführt werden. Als Lösungsmittel werden Mineral- oder Silikonöle benutzt. Nachteil dieser Lösungsmittel ist die heterogene Reaktionsmischung (3-phasig bestehend aus Lösungsmittel/Reaktionsgemisch/Katalysator).

Der vorliegenden Erfindung lag daher die Aufgabe zugrunde, den zuvor genannten Nachteilen abzuhelfen.

Demgemäß wurde ein neues und verbessertes Verfahren zur Herstellung von Indoleninen der allgemeinen Formel I

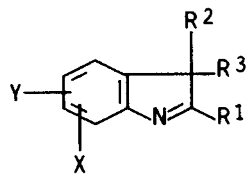 (I),

in der

| | |
|---|---|
| $R^1$, $R^2$, $R^3$ | unabhängig voneinander Wasserstoff, $C_1$- bis $C_{20}$-Alkyl, $c_2$- bis $C_{20}$-Alkenyl, $C_3$- bis $C_8$-Cycloalkyl, Aryl, $C_7$- bis $C_{20}$-Alkylphenyl, $C_7$- bis $C_{20}$-Phenalkyl oder $R^1$ und $R^2$ oder $R^1$ und $R^3$ gemeinsam eine gegebenenfalls durch $C_1$- bis $C_{12}$-Alkyl substituierte $C_3$- bis $C_8$-Alkylenkette, |
| X, Y | unabhängig voneinander Wasserstoff, $C_1$- bis $C_8$-Alkyl, $C_1$- bis $C_8$-Alkoxy, $C_2$- bis $C_8$-Alkoxyalkyl, $C_2$- bis $C_8$-Alkoxycarbonyl, $C_1$- bis $C_4$-Alkylsulfonyl, Halogen, Nitro oder Cyano |

bedeuten, durch Umsetzung von 4-Methylen-1,3-dioxolan-2-onen der allgemeinen Formel II

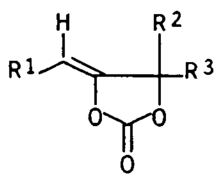 (II),

in der die Reste $R^1$, $R^2$ und $R^3$ die obengenannten Bedeutungen haben, mit Anilinen der allgemeinen Formel III

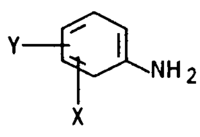 (III),

in der die Reste X und Y die obengenannten Bedeutungen haben, in Gegenwart von Lewissäuren und gegebenenfalls von Halogeniden der 1. oder 2. Hauptgruppe des Periodensystems der Elemente bei Temperaturen von 100 bis 350 °C und Drücken von 1 bis 50 bar gefunden, welches dadurch gekennzeichnet ist, daß man die Umsetzung in polaren Lösungsmitteln durchführt.

Das erfindungsgemäße Verfahren kann wie folgt durchgeführt werden:

Die Synthese von Indoleninen I nach dem erfindungsgemäßen Verfahren kann so durchgeführt werden, daß man die 4-Methylen-1,3-dioxolan-2-one II und die Aniline III in Gegenwart einer Lewissäure mit gegebenenfalls einem Halogenid der 1. und 2. Hauptgruppe der PSE gemeinsam in einem polaren Lösungsmittel vorlegt und erhitzt.

Ebenso ist es möglich, nur die Aniline III mit einem polaren Lösungsmittel, einer Lewissäure und einem Halogenid der 1. und 2. Hauptgruppe des PSE vorzulegen und bei der Reaktionstemperatur die 4-Methylen-1,3-dioxolan-2-one II hinzuzufügen.

Selbstverständlich ist es auch möglich, die Verbindungen der Formel II und III, sowie das polare Lösungsmittel und die Lewissäure und das Halogenid der 1. und 2. Hauptgruppe des PSE in kontinuierlicher Verfahrensweise zur Reaktion zu bringen. Vorteilhaft ist bei einer kontinuierlichen Reaktionsführung die Verwendung einer Rührkesselkaskade, insbesondere einer Zwei- oder Dreikesselkaskade.

Die Umsetzung der Verbindungen der Formel II mit Verbindungen der Formel III erfolgt unter $CO_2$- und $H_2O$-Abspaltung.

Zweckmäßigerweise führt man das erfindungsgemäße Verfahren bei Temperaturen von 100 bis 350 ° C, vorzugsweise bei 150 bis 300 ° C, besonders bevorzugt bei 200 bis 250 ° C, durch.

Bei Temperaturen größer 200 ° C wird die Reaktion im allgemeinen unter einem Druck von 1 bis 50 bar, vorzugsweise 1 bis 10 bar, besonders bevorzugt bei 2 bis 5 bar durchgeführt.

Bei der Reaktion entstehendes $CO_2$ und $H_2O$ werden über eine Druckhaltung aus dem System entfernt.

Als polare Lösungsmittel für das erfindungsgemäße Verfahren eignen sich hochsiedende, unter den Reaktionsbedingungen inerte Verbindungen, beispielsweise ein- bis vierfach am Stickstoff $C_1$-$C_8$-alkylsubstituierte Harnstoffe, wie N,N'-Dimethyl-, N,N'-Diethyl, N,N'-Dipropyl, N,N'-Dibutyl-, N,N,N',N'-Tetramethyl-, N,N,N',N'-Tetraethylharnstoff.

Der Harnstoff darf beispielsweise auch eine cyclische Struktur, wie Ethylen- oder Propylenharnstoff, sowie deren an den Stickstoffen ein- oder zweifach $C_1$-$C_8$-alkylsubstituierten Vertreter, wie N,N'-Dimethyl- oder N,N'-Diethylethylenharnstoff oder -propylenharnstoff, besitzen.

Erfindungsgemäße Lösungsmittel sind ebenfalls cyclische Lactame mit 5 bis 8 Kettengliedern, die gegebenenfalls $C_1$-$C_{12}$-Alkyl N-substituiert sein können. Beispielsweise seien Pyrrolidon, Piperidon und $\epsilon$-Caprolactam, sowie deren N-Methyl-, N-Ethyl, N-Propyl-, N-Butyl, N-Hexyl- oder N-Octyl-Derivate, insbesondere N-Methylpyrrolidon genannt.

Erfindungsgemäße Lösungsmittel sind auch aliphatische Polyalkohole mit 2 bis 5 Hydroxygruppen, wie z.B. Ethylenglykol, Propylenglykol, Glyzerin, Neopentylglykol oder Pentaerythrit, sowie Polyethylenglykole, wie Diethylenglykol, Triethylenglykol, Tetreaethylenglykol oder längerkettige Polyethylenglykole mit mittleren Molmassen von 250 bis 9000, insbesondere auch die an den End-OH-Gruppen mono- oder di- $C_1$-$C_5$-alkylierten Vertreter, wie z.B. Diethylenglykolmonomethylether, -dimethylether, -monoethylether, -diethylether, -monobutylether, -dibutylether, Triethylenglykolmonomethylether, -dimethylether, -monoethylether, -diethylether, -monobutylether, -dibutylether, Tetraethylenglykolmonomethylether, -dimethylether, -monoethylether, -diethylether, -monobutylether, -dibutylether oder Polyethylenglykole mittlerer Molmasse 250 bis 9000 als mono-, dimethyl-, ethyl-, butylether.

Als Lewissäuren für die Umsetzung eignen sich z.B. Aluminium-III-, Bor-III-, Eisen-III-, Titan-IV-, Zink-II-, Zinn-II- oder Zinn-IV-Salze, vorzugsweise deren Halogenide, wie Chloride, Bromide und Jodide, insbesondere Zink-II-chlorid.

Als Halogenide der 1. und 2. Hauptgruppe des PSE eignen sich beispielsweise LiCl, NaCl, KCl, LiBr, NaBr, KBr, LiJ, NaJ, KJ, $MgCl_2$, $CaCl_2$, $BaCl_2$, $MgBr_2$, $CaBr_2$, $BaBr_2$, $MgJ_2$, $CaJ_2$ und $BaJ_2$.

Die Substituenten $R^1$, $R^2$, $R^3$, X und Y in den Verbindungen I, II und III haben folgende Bedeutungen:

$R^1$, $R^2$, $R^3$ unabhängig voneinander

- Wasserstoff
- unverzweigtes oder verzweigtes $C_1$- bis $C_{20}$-Alkyl, vorzugsweise unverzweigtes oder verzweigtes $C_1$- bis $C_8$-Alkyl, wie n-Pentyl, iso-Pentyl, sec.-Pentyl, tert.-Pentyl, neo-Pentyl, 1,2-Dimethylpropyl, n-Hexyl, iso-Hexyl, sec.-Hexyl, n-Heptyl, iso-Heptyl, n-Octyl und iso-Octyl, besonders bevorzugt $C_1$- bis $C_4$-Alkyl wie Methyl, Ethyl, n-Propyl, iso-Propyl, n-Butyl, iso-Butyl, sec.-Butyl, tert.-Butyl,
- unverzweigtes oder verzweigtes $C_2$- bis $C_{20}$-Alkenyl, vorzugsweise unverzweigtes oder verzweigtes $C_2$- bis $C_8$-Alkenyl, wie Allyl, 2-Buten-1-yl, 4-Buten-2-yl, 4-Buten-1-yl, 2-Penten-1-yl und 2,2-Dimethylpenten-1-yl,
- $C_3$- bis $C_8$-Cycloalkyl, wie Cyclopropyl, Cyclobutyl, Cyclopentyl, Cyclohexyl, Cycloheptyl und Cyclooctyl,
- Aryl, wie Phenyl, 1-Naphthyl und 2-Naphthyl, bevorzugt Phenyl,
- $C_7$- bis $C_{20}$-Phenalkyl, bevorzugt $C_7$- bis $C_{10}$-Phenalkyl wie Benzyl, 1-Phenethyl und 2-Phenethyl,
- $C_7$- bis $C_{20}$-Alkylphenyl, wie 2-Methylphenyl, 4-Methylphenyl, 2-Ethylphenyl, 4-Ethylphenyl, 3-Methylphenyl, 2,4-Dimethylphenyl, 3,4-Dimethylphenyl und 3, 4, 5-Trimethylphenyl,

$R^1$ und $R^2$ oder $R^1$ und $R^3$

- gemeinsam
- eine $C_2$- bis $C_8$-Alkylenkette, wie $(CH_2)_2$, $(CH_2)_3$, $(CH_2)_4$, $(CH_2)_5$, $(CH_2)_6$, $(CH_2)_7$ und $(CH_2)_8$, bevorzugt $(CH_2)_4$, $(CH_2)_5$
- durch $C_1$- bis $C_{12}$-Alkyl ein- bis fünffach substituierte $C_2$- bis $C_8$-Alkylenkette, wie $CH_2(CH_3)CH$, $(CH_2)_2CH(CH_3)CH_2$,

X, Y

unabhängig voneinander

- Wasserstoff
- unverzweigtes oder verzweigtes $C_1$- bis $C_8$-Alkyl, wie n-Pentyl, iso-Pentyl, sec.-Pentyl, tert.-Pentyl, neo-Pentyl, 1,2-Dimethylpropyl, n-Hexyl, iso-Hexyl, sec.-Hexyl, n-Heptyl, iso-Heptyl, n-Octyl und iso-Octyl, bevorzugt $C_1$- bis $C_4$-Alkyl wie Methyl, Ethyl, n-Propyl, iso-Propyl, n-Butyl, iso-Butyl, sec.-Butyl und tert.-Butyl,
- $C_1$- bis $C_8$-Alkoxy, bevorzugt $C_1$- bis $C_4$-Alkoxy, wie Methoxy, Ethoxy, n-Propoxy, iso-Propoxy, n-Butoxy, iso-Butoxy, sec.-Butoxy und tert.-Butoxy,
- $C_2$- bis $C_8$-Alkoxyalkyl, bevorzugt $C_2$- bis $C_4$-Alkoxyalkyl, wie Methoxymethyl, Ethoxymethyl,
- $C_2$- bis $C_8$-Alkoxycarbonyl, bevorzugt $C_2$- bis $C_4$-Alkoxycarbonyl, wie Methoxycarbonyl, Ethoxycarbonyl,
- $C_1$- bis $C_4$-Alkylsulfonyl, wie Methylsulfonyl, Ethylsulfonyl,
- Halogen, wie Fluor, Chlor, Brom und Jod, bevorzugt Chlor und Brom,
- Nitro,
- Cyano.

Bevorzugte Indolenine I sind z.B.
2,3,3-Trimethyl-3(H)-indol
2,3,3,5-Tetramethyl-3(H)-indol
5-Chlor-2,3,3-trimethyl-3-(H)-indol

Die nach dem erfindungsgemäßen Verfahren herstellbaren Indolenine I sind Zwischenprodukte für die Herstellung von kationischen Cyanin- und Azacyaninfarbstoffen (Lit. Ullmann Band A16, 5. ed. S 487ff) sowie für die Photo-Industrie (z. B. US 3 865 837, Eastman Kodak; EP 288 083 Fuji Photo Film K.K.).

Beispiel 1

256 g 4,4-Dimethyl-5-methylen-1,3-dioxolan-2-on, 465 g Anilin, 20 g $ZnCl_2$, 12 g LiCl und 70 g N-Methylpyrrolidon werden zusammengemischt und 8 Stunden bei 200 °C gerührt. Parallel zur $CO_2$-Entwicklung wird $H_2O$ abgespaltet, das kontinuierlich aus dem Reaktionsgemisch abdestilliert. Nach der Reaktion werden bei der anschließenden Destillation 249 g (78 %) 2,3,3-Trimethyl-3(H)-indol, Sdp.: 10 Torr/110 °C erhalten.

Beispiel 2

465 g Anilin, 20 g $ZnCl_2$, 12 g LiCl und 70 g N-Methylpyrrolidon werden zum Rückfluß erhitzt und innerhalb 7 Stunden mit 256 g 4,4-Dimethyl-5-methylen-1,3-dioxolan-2-on versetzt. Nach dem Zutropfen wird 8 Stunden bei 200 °C gerührt und anschließend destilliert. Man erhält 251 g (79 %) 2,3,3-Trimethyl-3-(H)-indol, Sdp.: 10 Torr/110 °C.

Beispiel 3 bis 17

Die Durchführung erfolgte analog Beispiel 1 oder 2 mit 70 g der unten aufgeführten Lösungsmittel als Ersatz für N-Methylpyrrolidon.

| Lösungsmittel | analog Beispiel Nr. | Ausbeute [%] |
|---|---|---|
| Glyzerin | 1 | 63 |
| Glyzerin | 2 | 78 |
| Triethylenglykol | 1 | 73 |
| Triethylenglykol | 2 | 73 |
| Neopentylglykol | 2 | 74 |
| Pentaerythrit | 1 | 74 |
| Polyethylenglykol 600 | 2 | 67 |
| Polyethylenglykol 9000 | 2 | 69 |
| Diethylenglykoldibutylether | 1 | 79 |
| Polyethylenglykoldimethylether 250 | 1 | 80 |
| Polyethylenglykoldimethylether 500 | 1 | 79 |
| Polyethylenglykoldimethylether 1000 | 2 | 74 |
| N,N-Dimethylharnstoff | 2 | 73 |
| N,N-Dimethylethylenharnstoff | 1 | 76 |
| N,N-Dimethylpropylenharnstoff | 2 | 78 |

Beispiel 18 bis 29

Die Durchführung erfolgte analog Beispiel 1 oder 2 mit geänderter Lewissäure und Halogenide der 1. oder 2. Hauptgruppe des PSE (Verwendung identischer Mol-Äquivalente).

| Lewissäure | Halogenid der 1. oder 2. Hauptgruppe des PSE | analog Beispiel Nr. | Ausbeute [%] |
|---|---|---|---|
| $FeCl_3$ | LiCl | 1 | 45 |
| $AlCl_3$ | LiCl | 1 | 7 |
| $TiCl_4$ | LiCl | 1 | 8 |
| $SnCl_4$ | LiCl | 1 | 29 |
| $ZnCl_2$ | -- | 1 | 68 |
| $ZnCl_2$ | NaCl | 1 | 79 |
| $ZnCl_2$ | KCl | 1 | 78 |
| $ZnCl_2$ | $CaCl_2$ | 1 | 78 |
| $ZnCl_2$ | LiBr | 1 | 77 |
| $ZnCl_2$ | LiJ | 2 | 67 |
| $ZnBr_2$ | LiBr | 2 | 63 |
| $ZnJ_2$ | LIJ | 2 | 56 |

Beispiel 30 bis 33

Die Durchführung erfolgte analog Beispiel 1 mit geänderten Temperaturen, Drücken und Reaktionszeiten.

| Temperatur [° C] | Druck [bar] | Reaktionszeit [Std.] | Ausbeute [%] |
|---|---|---|---|
| 210 | 1 | 6 | 80 |
| 220 | 2 | 4 | 79 |
| 230 | 3 | 3 | 82 |
| 240 | 4 | 2 | 81 |

Beispiel 34

Die Durchführung erfolgte analog Beispiel 1 mit 535 g p-Toluidin. Man erhält nach Destillation 258 g (75 %) 2,3,3,5-Tetramethyl-3(H)-indol, Sdp.: 23 Torr/125 ° C.

## Patentansprüche

1. Verfahren zur Herstellung von Indoleninen der allgemeinen Formel I

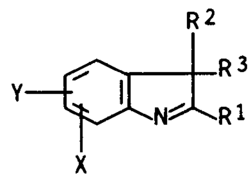

(I),

in der

$R^1$,$R^2$,$R^3$ unabhängig voneinander Wasserstoff, $C_1$- bis $C_{20}$-Alkyl, $C_2$- bis $C_{20}$-Alkenyl, $C_3$- bis $C_8$-Cycloalkyl, Aryl, $C_7$- bis $C_{20}$-Alkylphenyl, $C_7$- bis $C_{20}$-Phenalkyl oder $R^1$ und $R^2$ oder $R^1$ und $R^3$ gemeinsam eine gegebenenfalls durch $C_1$- bis $C_{12}$-Alkyl substituierte $C_3$- bis $C_8$-Alkylenkette,

X, Y unabhängig voneinander Wasserstoff, $C_1$- bis $C_8$-Alkyl, $C_1$- bis $C_8$-Alkoxy, $C_2$- bis $C_8$-Alkoxyalkyl, $C_2$- bis $C_8$-Alkoxycarbonyl, $C_1$- bis $C_4$-Alkylsulfonyl, Halogen, Nitro oder Cyano

bedeuten, durch Umsetzung von 4-Methylen-1,3-dioxolan-2-onen der allgemeinen Formel II

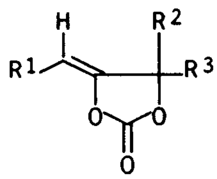

(II),

in der die Reste $R^1$, $R^2$ und $R^3$ die obengenannten Bedeutungen haben, mit Anilinen der allgemeinen Formel III

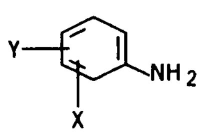

(III),

in der die Reste X und Y die obengenannten Bedeutungen haben, in Gegenwart von Lewissäuren und gegebenenfalls von Halogeniden der 1. oder 2. Hauptgruppe des Periodensystems der Elemente bei Temperaturen von 100 bis 350 ° C und Drücken von 1 bis 50 bar, dadurch gekennzeichnet, daß man die Umsetzung in polaren Lösungsmitteln durchführt.

2. Verfahren nach Anspruch 1, dadurch gekennzeichnet, daß man die Umsetzung bei Temperaturen von 200 bis 300 ° C durchführt.

3. Verfahren nach Anspruch 1, dadurch gekennzeichnet, daß man die Umsetzung bei Drücken von 2 bis 10 bar durchführt.

4. Verfahren nach Anspruch 1, dadurch gekennzeichnet, daß man als polare Lösungsmittel ein- bis vierfach durch $C_1$- bis $C_8$-Alkyl N-substituierte Harnstoffe, ein- oder zweifach durch $C_1$- bis $C_8$-Alkyl N-substituierte cyclische Harnstoffe, gegebenenfalls durch $C_1$- bis $C_{12}$-Alkyl N-substituierte cyclische Lactame mit 5 bis 8 Kettengliedern und/oder aliphatische Polyalkohole mit 2 bis 5 Hydroxygruppen und einem mittleren Molekulargewicht von 200 bis 10000 einsetzt.

5. Verfahren nach Anspruch 1, dadurch gekennzeichnet, daß man als polares Lösungsmittel ein- bis

vierfach durch $C_1$- bis $C_8$-Alkyl N-substituierte Harnstoffe einsetzt.

**6.** Verfahren nach Anspruch 1, dadurch gekennzeichnet, daß man als polares Lösungsmittel ein- oder zweifach durch $C_1$- bis $C_8$-Alkyl N-substituierte cyclische Harnstoffe einsetzt.

**7.** Verfahren nach Anspruch 1, dadurch gekennzeichnet, daß man als polares Lösungsmittel gegebenenfalls durch $C_1$- bis $C_{12}$-Alkyl N-substitierte cyclische Lactame mit 5 bis 8 Kettengliedern einsetzt.

**8.** Verfahren nach Anspruch 1, dadurch gekennzeichnet, daß man als polares Lösungsmittel N-Methylpyrrolidon einsetzt.

**9.** Verfahren nach Anspruch 1, dadurch gekennzeichnet, daß man als polares Lösungsmittel aliphatische Polyalkohole mit 2 bis 5 Hydroxygruppen und einem mittleren Molekulargewicht von 200 bis 10000 einsetzt.

Europäisches Patentamt

# EUROPÄISCHER RECHERCHENBERICHT

Nummer der Anmeldung

EP 92 10 5402

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| D,Y | EP-A-0 008 097 (BASF AG) 20. Februar 1980<br>* Ansprüche 1-4; Beispiel 3 *<br>--- | 1-9 | C07D209/08<br>C07D209/86<br>C07D209/94<br>C07D317/34<br>C07D317/44<br>C07D317/46<br>C07C211/46<br>C07C211/52<br>// C07D263/38<br>C07D263/52 |
| Y | KAGAKU (KYOTO)<br>Bd. 37, Nr. 11, 1982,<br>Seiten 858 - 861;<br>ODA RYOHEI: 'Review of new syntheses of indoles'<br>*Siehe speziell Punkt 6, Seite 860 f. ,<br>Indolenin Syntheseschema, Seite 861, oben*<br>--- | 1-9 | |
| A | EP-A-0 003 556 (BASF AG) 22. August 1979<br>* Ansprüche 1-4 *<br>--- | 1-9 | |
| A | DE-A-2 514 759 (SNAM PROGETTI S.P.A.) 9. Oktober 1975<br>*Ansprüche*<br>----- | 1-9 | |

| RECHERCHIERTE SACHGEBIETE (Int. Cl.5 ) |
|---|
| C07D<br>C07C |

Der vorliegende Recherchenbericht wurde für alle Patentansprüche erstellt

| Recherchenort | Abschlußdatum der Recherche | Prüfer |
|---|---|---|
| MUENCHEN | 17 AUGUST 1992 | HARTRAMPF G.W. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument